# EUROPÄISCHE PATENTANMELDUNG

(11) **EP 4 344 612 A1**
(43) Veröffentlichungstag der Anmeldung: **03.04.2024**
(21) Anmeldenummer: 23200557.9
(22) Anmeldetag: 28.09.2023
(51) Int. Cl.: A61B 3/00

(54) **VORRICHTUNG UND VERFAHREN ZUR VERARBEITUNG OPHTHALMOLOGISCHER MESSWERTE**

(30) Priorität: 29.09.2022 DE 202022105496 U; 03.11.2022 DE 102022129048
(71) Anmelder: medical future GmbH, 04279 Leipzig (DE)
(72) Erfinder: HUND, Alexander, 04416 Markkleeberg (DE)
(74) Vertreter: Hecht, Jan-David

(57) **Zusammenfassung**

Die vorliegende Erfindung betrifft eine Vorrichtung (12) und ein Verfahren zur Verarbeitung ophthalmologischer Messwerte, wobei eine Lösung geschaffen ist, die dahingehend Verbesserungen bringt, dass ophthalmologische Messgeräte (18, 20) von einem Benutzer nicht eigens bedient werden und deren Messergebnisse vom Benutzer in Bezug auf den Patienten auch nicht selbständig weiterverarbeitet werden müssen. Dabei ist die Benutzung dieser ophthalmologischen Untersuchungsgeräte (18, 20) auch einfacher und komfortabler.

## Beschreibung

Die vorliegende Erfindung betrifft eine Vorrichtung zur Verarbeitung ophthalmologischer Messwerte nach dem Oberbegriff von Anspruch 1 und ein Verfahren zur Verarbeitung ophthalmologischer Messwerte nach dem Oberbegriff von Anspruch 8.

Die Ophthalmologie oder Augenheilkunde ist ein Teilgebiet der Medizin, das die Lehre von der Erkennung (Diagnose), Behandlung (Therapie) und Vorbeugung (Prävention) von Erkrankungen des Auges und der umgebenden Strukturen umfasst.

Typische Bereiche der Ophthalmologie sind Erkrankungen der Cornea (Ulcera, Traumata, Degenerationserkrankungen), Erkrankungen der Augenvorderkammer (Glaukome, Iritis), Erkrankungen der Augenhinterkammer und der Linse (Katarakte, Linsenluxationen), Erkrankungen von Glaskörper und Gefäßhaut (Uveitis, Glaskörperblutungen), Erkrankungen der Retina (AMD, Gesichtsfeldausfälle, degenerative Erkrankungen, Farbenblindheiten), Erkrankungen des Sehnervs (Neuritiden, degenerative Erkrankungen, Kompressionssyndrome), Erkrankungen von Orbita und Tränendrüse (Infektionen, Blutungen, Sicca-Syndrome), Erkrankungen mit Einschränkungen der Refraktion (Myopie, Hyperopie, Presbyopie, Astigmatismus) und Erkrankungen der Lider (Gerstenkorn, Hagelkorn, Entropium, Ektropium).

Die wichtigsten ophthalmologischen Untersuchungsverfahren sind die folgenden:
- Fluoreszenzangiographie zur Gefäßdarstellung, vor allem der Netzhautgefäße, mittels Fluorescein
- Gonioskopie zur Untersuchung des Kammerwinkels
- Hornhauttopographie und -tomographie (Videokeratographie, Keratometrie) zur Darstellung der äußeren und inneren Hornhautform und des Schichtaufbaus
- Ophthalmoskopie zur direkten und indirekten Spiegelung des Augenhintergrundes
- Optische Kohärenztomographie zur Schichtdarstellung der Netzhaut, des Sehnervs, des Glaskörpers, der Aderhaut und des vorderen Augenabschnitts
- Orthoptik zur Untersuchung der Stellung und Beweglichkeit der Augen und des beidäugigen Sehens
- Pachymetrie zur Messung der Hornhautdicke zur Diagnostik des Glaukoms und für die refraktive Chirurgie
- Perimetrie zur Untersuchung des Gesichtsfeldes
- Refraktometrie zur Untersuchung auf optische Fehlsichtigkeiten (Ametropie)
- Retinale Gefäßanalyse zur Untersuchung kleiner Netzhautgefäße, die Rückschlüsse auf Gefäßveränderungen in anderen Organen (z. B. Herz) erlaubt
- Spaltlampenmikroskopie zur mikroskopischen Untersuchung der vorderen, mittleren und hinteren Augenabschnitte, ggf. unter Zuhilfenahme eines Kontaktglases oder einer Untersuchungslupe
- Tonometrie zur Messung des Augeninnendrucks
- Visusprüfung zur Ermittlung der Sehschärfe.

Für all diese Untersuchungsverfahren sind zumeist unterschiedliche Untersuchungsgeräte, wie Gonioskop, Keratometer, Lensmeter, Ophthalmoskop, Perimeter, Phoropter, Pachymeter, Refraktometer, Scheitelbrechwertmesser, Spaltlampenmikroskop und Tonometer erforderlich, die von einem Benutzer eigens bedient werden und deren Messergebnisse vom Benutzer in Bezug auf den Patienten selbständig weiterverarbeitet werden müssen. Bei den ophthalmologischen Untersuchungsgeräten könnte es sich auch um Kombinationsmessgeräte handeln, beispielsweise können Refraktometer, Keratometer, Tonometer und Pachymeter in einem Gerät kombiniert vorliegen.

Es ist daher Aufgabe der vorliegenden Erfindung, eine Lösung zu schaffen, die hier Verbesserungen bringt. Insbesondere soll die Benutzung der ophthalmologischen Untersuchungsgeräte einfacher und komfortabler werden.

Diese Aufgabe wird gelöst mit der erfindungsgemäßen Vorrichtung nach Anspruch 1 und dem erfindungsgemäßen Verfahren nach Anspruch 8 sowie dem Computerprogrammprodukt nach Anspruch 15. Vorteilhafte Weiterbildungen sind in den abhängigen Ansprüchen und in der nachfolgenden Beschreibung zusammen mit den Figuren angegeben.

Erfinderseits wurde erkannt, dass diese Aufgabe in überraschender Art und Weise dadurch besonders einfach gelöst werden kann, wenn die durch die ophthalmologischen Untersuchungsgeräte gewonnenen Messergebnisse direkt von dem Gerät an eine die verarbeitende Computereinrichtung weitergegeben werden, weil dann die Messergebnisse vom Benutzer nicht selbständig weiterverarbeitet werden müssen, sondern dies in Bezug auf den jeweiligen Patienten von der Computereinrichtung übernommen werden kann. Dadurch ist die Benutzung der ophthalmologischen Untersuchungsgeräte nicht nur einfacher und komfortabler, sondern insgesamt auch sicherer, weil Verarbeitungsfehler ausgeschlossen werden können. Weiterhin wird auch eine zentrale Datenverarbeitung ermöglicht, da die Vorrichtung nicht nur mit einem sondern auch mit mehreren ophthalmologischen Untersuchungsgeräten verbunden sein kann.

Die erfindungsgemäße Vorrichtung zur Verarbeitung ophthalmologischer Messwerte weist zumindest eine erste Schnittstelle zu einem ophthalmologischen Gerät, wobei die ophthalmologischen Messwerte mit dem ophthalmologischen Gerät ermittelt wurden, und zumindest eine zweite Schnittstelle zu einer Computereinrichtung und/oder einem Computernetzwerk auf, wobei die Vorrichtung angepasst ist, die Messwerte von der ersten Schnittstelle an die zweite Schnittstelle zu übertragen.

In einer vorteilhaften Weiterbildung ist vorgesehen, dass die erste Schnittstelle eine normierte Schnittstelle und/oder eine gerätespezifische zu einem ophthalmologischen Gerät ist, wobei das ophthalmologische Gerät bevorzugt ein Gerät ist aus der Gruppe umfassend: Gonioskop, Keratometer, Lensmeter, Ophthalmoskop, Perimeter, Phoropter, Pachymeter, Refraktometer, Scheitelbrechwertmesser, Spaltlampenmikroskop und Tonometer. Dann lassen sich beliebige ophthalmologische Geräte verwenden und damit die darauf basierenden Untersuchungsverfahren einfach und komfortabel durchführen und deren Ergebnisse weiterverarbeiten.

In einer vorteilhaften Weiterbildung ist vorgesehen, dass die zweite Schnittstelle eine Software-Schnittstelle oder eine Hardware-Schnittstelle ist, wobei die zweite Schnittstelle bevorzugt angepasst ist, zumindest einige Daten, insbesondere die ophthalmologischen Messwerte im csv-Format, GDT-Format, REST-Format, tmp-Format, txt-Format und/oder xml-Format zu übertragen. Dadurch kann die Weiterverarbeitung der Messergebnisse besonders einfach erfolgen.

In einer vorteilhaften Weiterbildung ist vorgesehen, dass die Vorrichtung angepasst ist, die von dem ophthalmologischen Gerät an die Vorrichtung übertragenen ophthalmologischen Messwerte in ein Standardformat, vorzugweise ein GDT-Format, REST-Format, tmp-Format, txt-Format und/oder xml-Format umzuwandeln. Dadurch wird eine besonders einfache Weiterverarbeitung der Messergebnisse ermöglicht.

In einer vorteilhaften Weiterbildung ist vorgesehen, dass die Vorrichtung angepasst ist, die ermittelten ophthalmologischen Messwerte auszuwerten, nummerisch anzuzeigen und/oder grafisch anzuzeigen, wobei die Vorrichtung bevorzugt angepasst ist, die ermittelten ophthalmologischen Messwerte im Standardformat auszuwerten, nummerisch anzuzeigen und/oder grafisch anzuzeigen. Dadurch kann eine gezielte bzw. tiefergehende Weiterverarbeitung auch ohne zusätzliche Programme, insbesondere Augeninformationssysteme oder elektronische Patientenakten erfolgen.

In einer vorteilhaften Weiterbildung ist vorgesehen, dass die Vorrichtung angepasst ist, die ermittelten ophthalmologischen Messwerte an ein Praxisverwaltungssystem, bevorzugt ein Patienteninformationssystem zu übergeben, wobei das Praxisverwaltungssystem insbesondere ein Augeninformationssystem umfasst. Dadurch können die Messwerte leicht in standardisierte Patientenverwaltungssysteme übernommen werden, was die Arbeitsabläufe bei Optikern sowie in Arztpraxen und Kliniken bedeutend erleichtert.

In einer vorteilhaften Weiterbildung ist vorgesehen, dass die Vorrichtung angepasst ist, die ermittelten ophthalmologischen Messwerte in einer Datenbank zu speichern. Dann kann eine Sicherung der Messwerte erfolgen, wodurch eine erhöhte Sicherheit besteht.

In einer vorteilhaften Weiterbildung ist vorgesehen, dass die Vorrichtung angepasst ist, Steuerbefehle an das ophthalmologische Gerät zu übermitteln, wobei die Steuerbefehle bevorzugt auf zuvor ermittelten ophthalmologischen Messwerten beruhen. Dann muss das ophthalmologische Gerät nicht eigens von einem Benutzer bedient werden, sondern kann quasi ferngesteuert werden, was den Komfort und die Sicherheit in der Benutzung des ophthalmologischen Geräts verbessert.

Vorzugsweise bestehen somit zumindest zwei ophthalmologischen Geräte, die mit der Vorrichtung in kommunikativer, bevorzugt bidirektionaler, Verbindung bestehen, wobei die Vorrichtung angepasst ist, die mit einem ophthalmologischen Gerät gewonnenen Messwerte als Basis für Steuerbefehle zu verwenden, die der Ansteuerung des zweiten ophthalmologischen Geräts dienen.

Außerdem kann dann die Untersuchung mit dem zweiten ophthalmologischen Gerät, das über Steuerbefehle angesteuert wird, die auf mit einem ersten ophthalmologischen Gerät gewonnenen Messwerten beruhen, wesentlich komfortabler, präziser und schneller erfolgen.

Beispielsweise könnten die von einem objektiven Refraktometer gewonnenen ophthalmologischen Messwerte zur Ansteuerung eines subjektiven Refraktometers (Phoropter) verwendet werden, wodurch an diesem subjektiven Refraktometer nur noch die Feineinstellung durch den Augenarzt vorgenommen und mit dem Patientenempfinden abgeglichen werden muss.

Andererseits könnten auch die von einem Lensmeter bzw. Scheitelbrechwertmesser gewonnenen ophthalmologischen Messwerte zur Ansteuerung eines subjektiven Refraktometers verwendet werden, wodurch an diesem subjektiven Refraktometer wiederum nur noch die Feineinstellung durch den Augenarzt vorgenommen und mit dem Patientenempfinden abgeglichen werden muss.

Weiterhin könnten die von einem Lensmeter bzw. Scheitelbrechwertmesser oder auch die von einem objektiven Refraktometer gewonnenen ophthalmologischen Messwerte zur Ansteuerung eines Perimeters verwendet werden.

Außerdem könnten die von einem Lensmeter bzw. Scheitelbrechwertmesser oder auch die von einem objektiven Refraktometer gewonnenen ophthalmologischen Messwerte zur Ansteuerung eines OCT-Untersuchungsgerätes ("Optical coherence tomography" - Optische Kohärenztomographie) verwendet werden.

Die Ansteuerung des zweiten ophthalmologischen Geräts durch mit einem ersten ophthalmologischen Gerät gewonnenen ophthalmologischen Messwerte kann automatisch erfolgen oder durch manuelle Freigabe durch einen Benutzer, beispielsweise einem Arzt, wobei der Benutzer diese Messwerte zuvor noch einmal auf Stichhaltigkeit überprüfen kann, bevor er die Messwerte zur Steuerung des zweiten ophthalmologischen Geräts freigibt. Hierzu kann beispielsweise das Anzeigen der ophthalmologischen Messwerte des ersten ophthalmologischen Geräts und das Drücken eines Knopfes (auf einem Bildschirm der Vorrichtung, beispielsweise auf einem Touch-Screen oder per Maus oder dgl., oder direkt an der Vorrichtung, beispielsweise über eine Tastatur) vorgesehen sein.

In einer vorteilhaften Weiterbildung ist vorgesehen, dass die Vorrichtung angepasst ist, basierend auf den ermittelten ophthalmologischen Messwerten Rezepte zu erstellen. Dadurch kann der Arbeitsablauf bei Optikern sowie in Arztpraxen und Kliniken noch weiter vereinfacht und damit komfortabler und zeitunaufwändiger gemacht werden.

In einer vorteilhaften Weiterbildung ist vorgesehen, dass die Vorrichtung eine dritte Schnittstelle aufweist, mit der ein Benutzer Daten eingeben kann, wobei die Daten bevorzugt solche sind aus der Gruppe umfassend: Steuerdaten für das ophthalmologische Gerät, ophthalmologische Messwerte und Patientendaten. Dann muss ein Benutzer nicht auf ein Patientenverwaltungssystem oder dgl. zugreifen, sondern beispielsweise Stammdaten zu Patienten direkt in die Vorrichtung eingeben.

Selbständiger Schutz wird beansprucht für das erfindungsgemäße System, das die erfindungsgemäße Vorrichtung und zumindest ein ophthalmologisches Gerät, bevorzugt zumindest zwei ophthalmologische Geräte umfasst.

Unabhängiger Schutz wird beansprucht für das erfindungsgemäße Verfahren zur Verarbeitung ophthalmologischer Messwerte, wobei eine erste Schnittstelle zu einem ophthalmologischen Gerät besteht und die ophthalmologischen Messwerte mit dem ophthalmologischen Gerät ermittelt wurden, und zumindest eine zweite Schnittstelle zu einer Computereinrichtung und/oder einem Computernetzwerk besteht, wobei die Messwerte von der ersten Schnittstelle an die zweite Schnittstelle übertragen werden.

In einer vorteilhaften Weiterbildung ist vorgesehen, dass die erfindungsgemäße Vorrichtung verwendet wird.

In einer vorteilhaften Weiterbildung ist vorgesehen, dass die erste Schnittstelle eine normierte Schnittstelle und/oder eine gerätespezifische zu einem ophthalmologischen Gerät ist, wobei das ophthalmologische Gerät bevorzugt ein Gerät ist aus der Gruppe umfassend: Gonioskop, Keratometer, Lensmeter, Ophthalmoskop, Perimeter, Phoropter, Pachymeter, Refraktometer, Scheitelbrechwertmesser, Spaltlampenmikroskop und Tonometer. Dann lassen sich beliebige ophthalmologische Geräte verwenden und damit die darauf basierenden Untersuchungsverfahren einfach und komfortabel durchführen und deren Ergebnisse weiterverarbeiten.

In einer vorteilhaften Weiterbildung ist vorgesehen, dass die zweite Schnittstelle eine Software-Schnittstelle oder eine Hardware-Schnittstelle ist, wobei die zweite bevorzugt Schnittstelle angepasst ist, zumindest einige Daten, insbesondere die ophthalmologischen Messwerte im csv-Format, GDT-Format, REST-Format, tmp-Format, txt-Format und/oder xml-Format zu übertragen. Dadurch kann die Weiterverarbeitung der Messergebnisse besonders einfach erfolgen.

In einer vorteilhaften Weiterbildung ist vorgesehen, dass die von dem ophthalmologischen Gerät an die Vorrichtung übertragenen ophthalmologischen Messwerte in ein Standardformat, vorzugweise ein GDT-Format, REST-Format, tmp-Format, txt-Format und/oder xml-Format umgewandelt werden. Dadurch wird eine besonders einfache Weiterverarbeitung der Messergebnisse ermöglicht.

In einer vorteilhaften Weiterbildung ist vorgesehen, dass die ermittelten ophthalmologischen Messwerte ausgewertet, nummerisch angezeigt und/oder grafisch angezeigt werden, wobei die ermittelten ophthalmologischen Messwerte im Standardformat ausgewertet, nummerisch angezeigt und/oder grafisch angezeigt werden. Dadurch kann eine gezielte bzw. tiefergehende Weiterverarbeitung auch ohne zusätzliche Programme, insbesondere Augeninformationssysteme oder elektronische Patientenakten erfolgen.

In einer vorteilhaften Weiterbildung ist vorgesehen, dass die ermittelten ophthalmologischen Messwerte an ein Praxisverwaltungssystem, bevorzugt ein Patienteninformationssystem übergeben werden, wobei das Patienteninformationssystem insbesondere ein Augeninformationssystem umfasst. Dadurch können die Messwerte leicht in standardisierte Praxisverwaltungssysteme übernommen werden, was die Arbeitsabläufe bei Optikern sowie in Arztpraxen und Kliniken bedeutend erleichtert.

In einer vorteilhaften Weiterbildung ist vorgesehen, dass die ermittelten ophthalmologischen Messwerte in einer Datenbank gespeichert werden. Dann kann eine Sicherung der Messwerte erfolgen, wodurch eine erhöhte Sicherheit besteht.

In einer vorteilhaften Weiterbildung ist vorgesehen, dass Steuerbefehle an das ophthalmologische Gerät übermittelt werden, wobei die Steuerbefehle bevorzugt auf zuvor ermittelten ophthalmologischen Messwerten beruhen. Dann muss das ophthalmologische Gerät nicht eigens von einem Benutzer bedient werden, sondern kann quasi ferngesteuert werden, was den Komfort und die Sicherheit in der Benutzung des ophthalmologischen Geräts verbessert.

Vorzugsweise bestehen somit zumindest zwei ophthalmologischen Geräte, die mit der Vorrichtung in kommunikativer Verbindung bestehen, wobei die mit einem ophthalmologischen Gerät gewonnenen Messwerte als Basis für Steuerbefehle verwendet werden, die der Ansteuerung des zweiten ophthalmologischen Geräts dienen.

Außerdem kann dann die Untersuchung mit dem zweiten ophthalmologischen Gerät, das über Steuerbefehle angesteuert wird, die auf mit einem ersten ophthalmologischen Gerät gewonnenen Messwerten beruhen, wesentlich komfortabler, präziser und schneller erfolgen.

Beispielsweise könnten die von einem objektiven Refraktometer gewonnenen ophthalmologischen Messwerte zur Ansteuerung eines subjektiven Refraktometers (Phoropter) verwendet werden, wodurch an diesem subjektiven Refraktometer nur noch die Feineinstellung durch den Augenarzt vorgenommen und mit dem Patientenempfinden abgeglichen werden muss.

Andererseits könnten auch die von einem Lensmeter bzw. Scheitelbrechwertmesser gewonnenen ophthalmologischen Messwerte zur Ansteuerung eines subjektiven Refraktometers verwendet werden, wodurch an diesem subjektiven Refraktometer wiederum nur noch die Feineinstellung durch den Augenarzt vorgenommen und mit dem Patientenempfinden abgeglichen werden muss.

Weiterhin könnten die von einem Lensmeter bzw. Scheitelbrechwertmesser oder auch die von einem objektiven Refraktometer gewonnenen ophthalmologischen Messwerte zur Ansteuerung eines Perimeters verwendet werden.

Außerdem könnten die von einem Lensmeter bzw. Scheitelbrechwertmesser oder auch die von einem objektiven Refraktometer gewonnenen ophthalmologischen Messwerte zur Ansteuerung eines OCT-Untersuchungsgerätes ("Optical coherence tomography" - Optische Kohärenztomographie) verwendet werden.

Die Ansteuerung des zweiten ophthalmologischen Geräts durch mit einem ersten ophthalmologischen Gerät gewonnenen ophthalmologischen Messwerte kann automatisch erfolgen oder durch manuelle Freigabe durch einen Benutzer, beispielsweise einem Arzt, wobei der Benutzer diese Messwerte zuvor noch einmal auf Stichhaltigkeit überprüfen kann, bevor er die Messwerte zur Steuerung des zweiten ophthalmologischen Geräts freigibt. Hierzu kann beispielsweise das Anzeigen der ophthalmologischen Messwerte des ersten ophthalmologischen Geräts und das Drücken eines Knopfes (auf einem Bildschirm der Vorrichtung, beispielsweise auf einem Touch-Screen oder per Maus oder dgl., oder direkt an der Vorrichtung, beispielsweise über eine Tastatur) vorgesehen sein.

In einer vorteilhaften Weiterbildung ist vorgesehen, dass basierend auf den ermittelten ophthalmologischen Messwerten Rezepte erstellt werden. Dadurch kann der Arbeitsablauf bei Optikern sowie in Arztpraxen und Kliniken noch weiter vereinfacht und damit komfortabler und zeitunaufwändiger gemacht werden.

In einer vorteilhaften Weiterbildung ist vorgesehen, dass eine dritte Schnittstelle verwendet wird, mit der ein Benutzer Daten eingeben kann, wobei die Daten bevorzugt solche sind aus der Gruppe umfassend: Steuerdaten für das ophthalmologische Gerät, ophthalmologische Messwerte und Patientendaten. Dann muss ein Benutzer nicht auf ein Patientenverwaltungssystem oder dgl. zugreifen, sondern beispielsweise Stammdaten zu Patienten direkt in die Vorrichtung eingeben.

Weiterhin kann die Erfindung in Gestalt eines Computerprogrammprodukts verwirklicht werden, das von einem computernutzbaren oder computerlesbaren Medium zugänglich ist und einen Programmcode für die Benutzung durch oder für die Benutzung in Verbindung mit einem Computer oder jedem Befehlsausführungssystem bereitgestellt ist. Daher wird auch selbständiger Schutz beansprucht für ein Computerprogrammprodukt, das auf einem für einen Computer lesbaren Medium gespeichert ist und für den Computer lesbare Programmmittel umfasst, die den Computer veranlassen, das erfindungsgemäße Verfahren auszuführen, wenn die Programmmittel auf dem Computer ausgeführt werden.

Für die Zwecke dieser Beschreibung können computernutzbare oder computerlesbare Medien alle Einrichtungen oder Vorrichtungen sein, die das Programm für die Benutzung durch oder die Benutzung in Verbindung mit dem Befehlsausführungssystem, der Vorrichtung oder der Einrichtung enthalten, speichern, kommunizieren, verbreiten oder transportieren. Dabei können auch mobile Kommunikationsmittel, beispielsweise Mobiltelefone,

Tabletcomputer und dgl. eingesetzt werden.

Das Medium kann ein elektronisches, magnetisches, optisches, elektromagnetisches, Infrarot- oder Halbleitersystem (oder Vorrichtung oder Einrichtung) sein oder ein Ausbreitungsmedium. Beispiele eines computerlesbaren Mediums umfassen einen Halbleiter oder

Feststoffspeicher, Magnetband, eine entfernbare Computerdiskette, einen Random Access Memory (RAM), einen Read-only Memory (ROM), eine feste magnetische Disk und eine optische Disk. Gegenwärtige Beispiele von optischen Disks umfassen Compactdisk-Read-only Memory (CD-ROM), Compactdisk-Read/Write (CD-R/W) und DVD.

Ein Datenverarbeitungssystem, das geeignet ist, den Programmcode zu speichern und/oder auszuführen, umfasst wenigstens einen Prozessor, der direkt oder indirekt mit zumindest einem Speicherelement durch einen Systembus verbunden ist. Das Speicherelement kann lokalen Speicher umfassen, der während der aktuellen Ausführung des Programmcodes tätig wird, Massenspeicher und Pufferspeicher, der eine temporäre Speicherung von wenigstens einigen Programmcodes bereitstellt, um die Anzahl an Abrufen des Codes vom Massenspeicher während der Ausführung zu reduzieren.

Eingabe/Ausgabe- oder I/O-Einrichtungen, die Tastaturen, Displays, Zeigeeinrichtungen etc. umfassen können, jedoch nicht darauf limitiert sind, können mit dem System entweder direkt oder durch zwischen geschaltete I/O-Controller an das System angekoppelt sein. Netzwerkadapter können ebenfalls mit dem System verbunden sein, um zu ermöglichen, dass das Daten verarbeitende System mit anderen Datenverarbeitungssystemen oder entfernten Druckern oder Speichereinrichtungen durch zwischengeschaltete private oder öffentliche Netzwerke angekoppelt wird. Modems, Kabelmodems oder Ethernet-Karten sind in diesem Zusammenhang nur einige Beispiele der gegenwärtig verfügbaren Typen von Netzwerkadaptern.

Die Merkmale und weitere Vorteile der vorliegenden Erfindung werden im Folgenden anhand der Beschreibung eines bevorzugten Ausführungsbeispiels im Zusammenhang mit der einzigen Figur deutlich werden. Dabei zeigt rein schematisch:

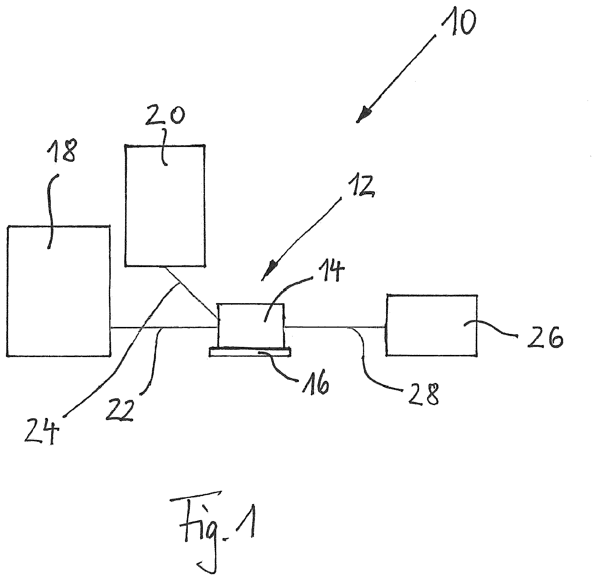

- Fig. 1: die erfindungsgemäße Vorrichtung im Rahmen ihrer Benutzung.

In Fig. 1 ist ein ophthalmologisches System 10 dargestellt, das die erfindungsgemäße Vorrichtung 12 aufweist, die eine Anzeigeeinheit 14 und eine Eingabeeinheit in Form einer Tastatur 16 umfasst, sowie zwei ophthalmologische Untersuchungsgeräte 18, 20, die jeweils über eine Datenleitung 22, 24 mit der Vorrichtung 12 kommunikationstechnisch verbunden sind, wobei die Vorrichtung 12 selbst mit einem Computernetzwerk 26 über die Datenleitung 28 kommunikationstechnisch verbunden ist.

Das erste ophthalmologische Untersuchungsgerät 18 ist beispielsweise ein Phoropter und das zweite ophthalmologische Untersuchungsgerät 20 ein Tonometer. Es könnte sich auch um andere ophthalmologische Untersuchungsgeräte handeln, wobei diese auch als Kombinationsmessgeräte, beispielsweise mit Refraktometer, Keratometer, Tonometer und Pachymeter vorliegen können.

Die erfindungsgemäße Vorrichtung 12 kann beispielsweise eine Computereinrichtung, wie ein Laptop oder dgl. sein. Anstelle dieser körperlich vorhandenen Vorrichtung könnte auch ein Computerprogrammprodukt vorliegen, das auf einer solchen Computereinrichtung ausgeführt wird, die insbesondere auch Teil des Computernetzwerkes 26 sein kann.

Das Computernetzwerk 26 ist beispielsweise als ein Ethernet-Netzwerk ausgebildet, in dem Praxisverwaltungssystem, bevorzugt ein Patienteninformationssystem, insbesondere mit einem Augeninformationssystem, beides in Softwareform, läuft.

Die Computereinrichtung 12 weist zwei erste Schnittstellen (nicht gezeigt) zur Anbindung der Datenleitungen 22, 24 auf, wobei diese Schnittstellen üblicherweise proprietäre Schnittstellen sind, die auf den jeweiligen Gerätetyp abgestimmt sind.

An der Vorrichtung 12 können nicht nur zwei sondern einen Vielzahl von ophthalmologischen Untersuchungsgeräten angeschlossen und über die Vorrichtung 12 bedient und ausgelesen werden. Außerdem können ophthalmologische Untersuchungsgeräte auch über das Computernetzwerk 26 mit der Vorrichtung 12 verbunden werden. Dies geht sehr leicht mit ophthalmologischen Untersuchungsgeräten, die eine RJ45-Schnittstelle aufweisen, weil sich diese dann standardmäßig an moderne Computernetzwerke 26, die ebenfalls solche Schnittstellen aufweisen, anschließen lassen.

Die Vorrichtung 12 ist so - beispielsweise durch eine entsprechende Software-Anwendung - ausgebildet, dass sie die von den einzelnen ophthalmologischen Untersuchungsgeräten 18, 20 an die Vorrichtung 12 übermittelten Daten gerätespezifisch in xml- oder txt-Format konvertiert werden, wenn sie nicht sowieso schon in diesem Format vom ophthalmologischen Untersuchungsgeräte 18, 20 übermittelt werden. Diese xml- oder tyt-Daten werden in der Vorrichtung 12 abgespeichert.

Diese konvertierten Daten können dann untersuchungsspezifisch numerisch und/oder grafisch angezeigt und ausgewertet werden, wozu die Vorrichtung 12 verschiedene Anzeige- bzw. Auswertemodule bereitstellt, die auf der Anzeigeeinheit 14 je nach Anwendungsfall angezeigt und bedient werden können. Dadurch kann der Benutzer alle ermittelten Messwerte auf einer übersichtlichen und einheitlichen Oberfläche darstellen ohne dabei auf proprietäre Software der einzelnen ophthalmologischen Untersuchungsgeräte oder andere Software zurückgreifen zu müssen.

Die Vorrichtung 12 selbst oder auch das Computernetzwerk 26 weisen eine SQL-Datenbank (nicht gezeigt) auf. Dank dieser SQL-Datenbank erfolgt eine schnelle und zentrale Datenspeicherung über die Vorrichtung 12 selbst, wodurch darüber hinaus auch eine sicherere automatisierbare Backup-Funktion unterstützt wird. In der Datenbank können auch die ermittelten Werte abgespeichert werden.

Weiterhin konvertiert die Vorrichtung 12 die übermittelten Daten direkt und/oder die konvertierten Daten in ein neues Format, nämlich entweder das GDT-Format (GDT steht für Gerätedatenträger) oder das REST-Format und übermittelt die Daten dann an das im Netzwerk 26 eingerichtete Patienteninformationssystem.

Diese Konvertierung erfolgt unter Berücksichtigung des ursprünglichen Dateiformates sowie des Schnittstellenformates, der Modellnummer und der gemessenen Daten des ophthalmologischen Untersuchungsgeräts.

Die an das Patienteninformationssystem übermittelten Daten erzeugen darin einen Karteikarteneintrag für den jeweiligen Patienten, wobei sie ggf. auch den neuen Patienten gleich mit anlegen, wenn dieser im Patienteninformationssystem noch nicht vorhanden ist. Aufgrund des normierten Datenformats (GDT oder REST) ist es dabei völlig egal welches Patienteninformationssystem verwendet wird.

Der Benutzer kann außerdem bestimmte Messwerte zur Ansteuerung der ophthalmologischen Messgeräte 18, 20 verwenden. Beispielsweise könnte der Benutzer mit der Vorrichtung 12 an den Phoropter 18 für einen Patienten abgespeicherte Werte für die subjektive Refraktion senden, um diesen schon einmal grob in Bezug auf die Anforderungen des Patienten einzustellen. Diese abgespeicherten Werte hätte der Benutzer beispielsweise vorab mit einem Autorefraktometer bei diesem Patienten ermitteln und über die Vorrichtung 12 abspeichern können.

Die Übermittlung der Werte an andere Geräte erfolgt über eine Schnittstelle, z.B. COM Phoropter / LAN oder mittels der GDT Softwarebasis.

Solche Messwerte zur Ansteuerung des Phoropters 18 hätte der Benutzer aber auch von einem Lensmeter/Scheitelbrechwertmesser (nicht gezeigt) gewinnen können.

Durch diese Ansteuerung des Phoropters 18 mit zuvor gewonnenen ophthalmologischen Messwerten kann der Komfort und die Sicherheit in der Benutzung des Phoropters 18 wesentlich verbessert werden. Insbesondere die Untersuchung mit dem Phoropter 18 wesentlich präziser und schneller, wobei durch den Augenarzt an dem Phoropter 18 nur noch die Feineinstellung vorgenommen und mit dem Patientenempfinden abgeglichen werden muss. Alternativ bzw. zusätzlich könnten beispielsweise auch die von einem Lensmeter bzw. Scheitelbrechwertmesser oder auch die von einem objektiven Refraktometer gewonnenen ophthalmologischen Messwerte zur Ansteuerung eines Perimeters oder eines OCT-Untersuchungsgerätes ("Optical coherence tomography" - Optische Kohärenztomographie) verwendet werden.

Außerdem können die ophthalmologischen Messwerte nicht nur an das Patienteninformationssystem übertragen werden, sondern es könnte beispielsweise auch gleich vor Ort ein Formular ausgedruckt werden, insbesondere eine Sehhilfenverordnung, oder die Werte können für den Patienten zum Mitnehmen übersichtlich ausgedruckt werden.

Aus der vorstehenden Darstellung ist deutlich geworden, dass mit der vorliegenden Erfindung eine Lösung geschaffen ist, die dahingehend Verbesserungen bringt, dass ophthalmologische Messgeräte von einem Benutzer nicht eigens bedient werden und deren Messergebnisse vom Benutzer in Bezug auf den Patienten auch nicht selbständig weiterverarbeitet werden müssen. Dabei ist die Benutzung dieser ophthalmologischen Untersuchungsgeräte einfacher und komfortabler. Die Erfindung kann sowohl in Form einer Vorrichtung, eines Verfahrens, aber auch in Form eines Computerprogrammprodukts und damit einer Software verwirklicht werden, die sowohl auf einen Einzelrechner als auch in einem Computernetzwerk operiert, wodurch ophthalmologische Messgeräte an ein Praxisverwaltungssystem oder eine andere datenverarbeitende Software (beispielsweise für Optiker) angebunden werden können. Dies umfasst beispielsweise Scheitelbrechwertmesser, objektive und subjektive Refraktion inklusive Akkomodationsbreiten- und Keratomiemessungen, Phoroptoren und Tonometrie mit Pachymetriemessungen. Gleichfalls können Visusmesswerte bei spezifischen Messungen mitverarbeitet werden. Die Übertragung erfolgt dabei vorzugsweise im standardisierten GDT-Format oder nach dem REST / API Verfahren an das Praxisverwaltungssystem. Die analysierten Daten werden untersuchungsspezifisch konvertiert und eingepflegt. Die Integration über herstellerspezifische Protokolle umfasst nicht nur eine Vielzahl von neuen Geräten (z.B. der Firmen Nidek, Topcon und Shin Nippon), sondern auch ältere Geräte, die beispielsweise den Ethernet Standard unterstützen. Dabei können mehrere ophthalmologische Messgeräte an die Vorrichtung bzw. mit der Software verbunden werden. Dank des modularen Aufbaus im Rahmen der Anzeige der Vorrichtung bzw. der Software können die übermittelten Werte und Messungen effektiv und schnell ausgewertet sowie numerisch und grafisch dargestellt werden.

Die jetzt mit der Anmeldung und auch die später eingereichten Ansprüche sind ohne Präjudiz für die Erzielung weitergehenden Schutzes.

Sollte sich hier bei näherer Prüfung, insbesondere auch des einschlägigen Standes der Technik, ergeben, dass das eine oder andere Merkmal für das Ziel der Erfindung zwar günstig, nicht aber entscheidend wichtig ist, so wird selbstverständlich schon jetzt eine Formulierung angestrebt, die ein solches Merkmal, insbesondere im Hauptanspruch, nicht mehr aufweist. Auch eine solche Unterkombination ist somit von der Offenbarung dieser Anmeldung abgedeckt.

Die in den abhängigen Ansprüchen angeführten Rückbeziehungen weisen auf die weitere Ausbildung des Gegenstandes des Hauptanspruches durch die Merkmale des jeweiligen Unteranspruches hin. Jedoch sind diese nicht als ein Verzicht auf die Erzielung eines selbständigen, gegenständlichen Schutzes für die Merkmale der rückbezogenen Unteransprüche zu verstehen.

Es ist weiter zu beachten, dass die in den verschiedenen Ausführungsformen beschriebenen und in den Figuren gezeigten Ausgestaltungen und Varianten der Erfindung beliebig untereinander kombinierbar sind. Dabei sind einzelne oder mehrere Merkmale beliebig gegeneinander austauschbar. Diese Merkmalskombinationen sind ebenso mit offenbart.

Merkmale, die nur in der Beschreibung offenbart wurden oder auch Einzelmerkmale aus Ansprüchen, die eine Mehrzahl von Merkmalen umfassen, können jederzeit als von erfindungswesentlicher Bedeutung zur Abgrenzung vom Stande der Technik in den oder die unabhängigen Anspruch/Ansprüche übernommen werden, und zwar auch dann, wenn solche Merkmale im Zusammenhang mit anderen Merkmalen erwähnt wurden beziehungsweise im Zusammenhang mit anderen Merkmalen besonders günstige Ergebnisse erreichen.

Somit können alle in der allgemeinen Beschreibung der Erfindung, der Beschreibung der Ausführungsbeispiele, den nachfolgenden Ansprüchen und in den Figuren dargestellten Merkmale sowohl einzeln als auch in beliebiger Kombination miteinander erfindungswesentlich sein. Diese Merkmale bzw. Merkmalskombinationen können jeweils eine selbständige Erfindung begründen, deren Inanspruchnahme sich ausdrücklich vorbehalten wird. Dabei müssen einzelne Merkmale aus der Beschreibung eines Ausführungsbeispiels nicht zwingend mit ein oder mehreren oder allen anderen in der Beschreibung dieses Ausführungsbeispiels angegebenen Merkmale kombiniert werden, diesbezüglich ist jede Unterkombination ausdrücklich mit offenbart. Außerdem können gegenständliche Merkmale einer Vorrichtung umformuliert auch als Verfahrensmerkmale Verwendung finden und Verfahrensmerkmale können umformuliert als gegenständliche Merkmale einer Vorrichtung Verwendung finden. Eine solche Umformulierung ist somit automatisch mit offenbart.

### Bezugszeichenliste

- 10: ophthalmologisches System
- 12: erfindungsgemäße Vorrichtung zur Verarbeitung ophthalmologischer Mess-werte
- 14: Anzeigeeinheit
- 16: Eingabeeinheit, Tastatur
- 18: erstes ophthalmologisches Untersuchungsgerät, Phoropter
- 20: zweites ophthalmologisches Untersuchungsgerät, Tonometer
- 22, 24: Datenleitungen zur Verbindung der Vorrichtung 12 mit den ophthalmologischen Untersuchungsgeräten 18, 20
- 26: Computernetzwerk
- 28: Datenleitung zwischen Vorrichtung 12 und Computernetzwerk

## Patentansprüche

1. Vorrichtung (12) zur Verarbeitung ophthalmologischer Messwerte mit zumindest einer ersten Schnittstelle zu einem ophthalmologischen Gerät (18, 20), wobei die ophthalmologischen Messwerte mit dem ophthalmologischen Gerät (18, 20) ermittelt wurden, und zumindest einer zweiten Schnittstelle zu einer Computereinrichtung und/oder einem Computernetzwerk (26), wobei die Vorrichtung (12) angepasst ist, die Messwerte von der ersten Schnittstelle an die zweite Schnittstelle zu übertragen.

2. Vorrichtung (12) nach Anspruch 1, **dadurch gekennzeichnet, dass** die Vorrichtung (12) angepasst ist, Steuerbefehle an das ophthalmologische Gerät zu übermitteln, wobei die Steuerbefehle bevorzugt auf zuvor ermittelten ophthalmologischen Messwerten beruhen.

3. Vorrichtung (12) nach Anspruch 1 oder 2, **dadurch gekennzeichnet,**
**dass** die erste Schnittstelle eine normierte Schnittstelle und/oder eine gerätespezifische zu einem ophthalmologischen Gerät (18, 20) ist, wobei das ophthalmologische Gerät bevorzugt ein Gerät ist aus der Gruppe umfassend: Gonioskop, Keratometer, Lensmeter, Ophthalmoskop, Perimeter, Phoropter (18), Pachymeter, Refraktometer, Scheitelbrechwertmesser, Spaltlampenmikroskop und Tonometer (20), und/oder **dass** die Vorrichtung (12) angepasst ist, die ermittelten ophthalmologischen Messwerte an ein Praxisverwaltungssystem, bevorzugt ein Patienteninformationssystem zu übergeben, wobei das Praxisverwaltungssystem insbesondere ein Augeninformationssystem umfasst und/oder
**dass** die Vorrichtung (12) angepasst ist, die ermittelten ophthalmologischen Messwerte in einer Datenbank zu speichern und/oder
**dass** die Vorrichtung (12) angepasst ist, basierend auf den ermittelten ophthalmologischen Messwerten Rezepte zu erstellen.

4. Vorrichtung (12) nach einem der vorherigen Ansprüche, **dadurch gekennzeichnet, dass** die zweite Schnittstelle eine Software-Schnittstelle oder eine Hardware-Schnittstelle ist, wobei die zweite bevorzugt Schnittstelle angepasst ist, zumindest einige Daten, insbesondere die ophthalmologischen Messwerte im csv-Format, GDT-Format, REST-Format, tmp-Format, txt-Format und/oder xml-Format zu übertragen.

5. Vorrichtung (12) nach einem der vorherigen Ansprüche, **dadurch gekennzeichnet, dass** die Vorrichtung (12) angepasst ist, die von dem ophthalmologischen Gerät (18, 20) an die Vorrichtung (12) übertragenen ophthalmologischen Messwerte in ein Standardformat, vorzugweise ein GDT-Format, REST-Format, tmp-Format, txt-Format und/oder xml-Format umzuwandeln.

6. Vorrichtung (12) nach einem der vorherigen Ansprüche, **dadurch gekennzeichnet, dass** die Vorrichtung (12) angepasst ist, die ermittelten ophthalmologischen Messwerte auszuwerten, nummerisch anzuzeigen und/oder grafisch anzuzeigen, wobei die Vorrichtung (12) bevorzugt angepasst ist, die ermittelten ophthalmologischen Messwerte im Standardformat nach Anspruch 4 auszuwerten, nummerisch anzuzeigen und/oder grafisch anzuzeigen.

7. Vorrichtung (12) nach einem der vorherigen Ansprüche, **dadurch gekennzeichnet, dass** die Vorrichtung (12) eine dritte Schnittstelle aufweist, mit der ein Benutzer Daten eingeben kann, wobei die Daten bevorzugt solche sind aus der Gruppe umfassend: Steuerdaten für das ophthalmologische Gerät, ophthalmologische Messwerte und Patientendaten.

8. Verfahren zur Verarbeitung ophthalmologischer Messwerte, wobei eine erste Schnittstelle zu einem ophthalmologischen Gerät (18, 20) besteht und die ophthalmologischen Messwerte mit dem ophthalmologischen Gerät (18, 20) ermittelt wurden, und zumindest eine zweite Schnittstelle zu einer Computereinrichtung und/oder einem Computernetzwerk (26) besteht, wobei die Messwerte von der ersten Schnittstelle an die zweite Schnittstelle übertragen werden.

9. Verfahren nach Anspruch 8, **dadurch gekennzeichnet, dass** Steuerbefehle an das ophthalmologische Gerät (18, 20) übermittelt werden, wobei die Steuerbefehle bevorzugt auf zuvor ermittelten ophthalmologischen Messwerten beruhen.

10. Verfahren nach Anspruch 8 oder 9, **dadurch gekennzeichnet,**
**dass** die ermittelten ophthalmologischen Messwerte an ein Praxisverwaltungssystem, bevorzugt ein Patienteninformationssystem übergeben werden, wobei das Praxisverwaltungssystem insbesondere ein Augeninformationssystem umfasst und/oder
**dass** die ermittelten ophthalmologischen Messwerte in einer Datenbank gespeichert werden und/oder
**dass** basierend auf den ermittelten ophthalmologischen Messwerten Rezepte erstellt werden dass die erste Schnittstelle eine normierte Schnittstelle und/oder eine gerätespezifische zu einem ophthalmologischen Gerät (18, 20) ist, wobei das ophthalmologische Gerät bevorzugt ein Gerät ist aus der Gruppe umfassend: Gonioskop, Keratometer, Ophthalmoskop, Perimeter, Phoropter (18), Pachymeter, Refraktometer, Scheitelbrechwertmesser, Spaltlampenmikroskop und Tonometer (20), und/oder
**dass** die Vorrichtung nach einem der Ansprüche 1 bis 7 verwendet wird.

11. Verfahren nach einem der Ansprüche 8 bis 10, **dadurch gekennzeichnet, dass** die zweite Schnittstelle eine Software-Schnittstelle oder eine Hardware-Schnittstelle ist, wobei die zweite bevorzugt Schnittstelle angepasst ist, zumindest einige Daten, insbesondere die ophthalmologischen Messwerte im GDT-Format, REST-Format, tmp-Format, txt-Format und/oder xml-Format zu übertragen.

12. Verfahren nach einem der Ansprüche 8 bis 11, **dadurch gekennzeichnet, dass** die von dem ophthalmologischen Gerät (18, 20) an die Vorrichtung (12) übertragenen ophthalmologischen Messwerte in ein Standardformat, vorzugweise ein GDT-Format, REST-Format, tmp-Format, txt-Format und/oder xml-Format umgewandelt werden.

13. Verfahren nach einem der Ansprüche 8 bis 12, **dadurch gekennzeichnet, dass** die ermittelten ophthalmologischen Messwerte ausgewertet, nummerisch angezeigt und/oder grafisch angezeigt werden, wobei die ermittelten ophthalmologischen Messwerte im Standardformat nach Anspruch 11 ausgewertet, nummerisch angezeigt und/oder grafisch angezeigt werden.

14. Verfahren nach einem der Ansprüche 8 bis 13, **dadurch gekennzeichnet, dass** eine dritte Schnittstelle verwendet wird, mit der ein Benutzer Daten eingeben kann, wobei die Daten bevorzugt solche sind aus der Gruppe umfassend: Steuerdaten für das ophthalmologische Gerät, ophthalmologische Messwerte und Patientendaten.

15. Computerprogrammprodukt, das auf einem für einen Computer lesbaren Medium gespeichert ist, umfassend für den Computer lesbare Programmmittel, die den Computer veranlassen, ein Verfahren nach einem der Ansprüche 8 bis 14 auszuführen, wenn die Programmmittel auf dem Computer ausgeführt werden.
